# EUROPEAN PATENT APPLICATION

(11) **EP 4 497 816 A1**
(43) Date of publication of application: **29.01.2025**
(21) Application number: 22932567.5
(22) Date of filing: 22.03.2022
(51) Int. Cl.: C12M 3/08

(54) **CLOSED CELL SEPARATION OPERATION APPARATUS**

(71) Applicant: Duogenic Stemcells Corporation, Taichung City, Taiwan (TW)
(72) Inventor: SU, Hong Lin, Taichung City, Taiwan (TW); SHEN, Ching I, Taichung City, Taiwan (TW); WANG, Fu Hui, Taichung City, Taiwan (TW); HSIEH, Chia Ying, Taichung City, Taiwan (TW)
(74) Representative: Viering, Jentschura & Partner mbB Patent- und Rechtsanwälte
(86) International application number: PCT/CN2022/082170
(87) International publication number: WO 2023/178498

(57) **Abstract**

A closed cell separation operation apparatus includes a first tube body (10) having a vent and an inlet, a second tube body (20) having a vent (131) and an inlet and a connecting pipe (24), connecting the first tube body (10) and a second tube body (20); the vent (131) of the first tube body (10) allows air to enter and exit the first tube body (10) and the inlet (132) of the first body (10) allows a fluid from an external fluid source to enter the first tube body (10); and the first tube body (10) having an outlet (121); the vent (231) of the second tube body (20) allows air to enter and exit the second tube body (20) and the inlet (232) of the second tube body (20) allows at least one part of the fluid from the outlet (121) of the first tube body (10) to enter the second tube body (20) and the second tube body (20) having an outlet (221). The closed cell separation operation apparatus provides a closed, contamination-free environment for both centrifugation and post-centrifugation supernatant separation.

## Description

### BACKGROUND OF THE INVENTION

### 1. Field of the Invention:

The present invention relates to centrifugal separation, and more particularly to a closed cell separation operation apparatus with a design that provides a closed, contamination-free environment for both centrifugation and post-centrifugation supernatant separation. The closed cell separation operation apparatus is particularly useful in, for example, cell separation and isolation.

### 2. Description of the Related Art:

Centrifugation has been extensively used in cell-related studies and assays to sort cells in such as bone marrow aspirate concentrate (BMAC), cerebrospinal fluid (CSF), peripheral blood, urine, ascites, cell culture media, etc.

As an essential tool for centrifugal separation, a conventional centrifuge tube is made as a capped test tube. Examples may be those traded under the name of Nunc (Thermo Fisher Scientific, the US). In use of such an existing centrifuge tube, a fluid to be subject to centrifugal processing is transferred into it, and then the centrifuge tube is sealed and placed into a centrifuge for processing. After centrifugation, for removing the resulting supernatant (e.g., plasma for whole blood centrifugation) and harvesting the sediment (e.g., blood cells for whole blood centrifugation), or vice versa, an operator has to separate the two parts by unsealing the tube and transferring the supernatant to a spear vessel. However, this operation unavoidably exposes the supernatant and sediment to the ambient environment and brings about risks of contamination. One solution to address the concern is to perform the whole operation in a clean room or a biosafety cabinet (BSC), which is usually costly and relatively immobile, thus unsuitable for clinical use.

### SUMMARY OF THE INVENTION

### TECHNICAL PROBLEM

Another problem of the conventional practice for post-centrifugation supernatant separation is that spill tends to happen in the process of transferring the content of the centrifuge tube and reduce the volume of the available specimen. This may have adverse effects on the intended study or assay, or in the worst case, makes it impossible to perform the intended study or assay.

Hence, there is a need for a novel apparatus that allows post-centrifugation supernatant separation to be performed efficiently and effusively in a contamination-free manner while eliminating the risks of spill and the needs for costly laboratory equipment.

### SOLUTION TO PROBLEMS

### TECHNICAL SOLUTION

In view of the aforementioned need, the primary objective of the present invention is to provide a closed cell separation operation apparatus that is designed to provide a closed, contamination-free environment for both centrifugation and post-centrifugation supernatant separation while eliminating the risks of spills and the needs for costly laboratory equipment.

In order to achieve this and other objectives of the present invention, the disclosed closed cell separation operation apparatus comprises: a first tube body, being a cylinder-like container defined by a peripheral wall, the first tube body having a closed top that sealably communicates with the exterior through a vent and an inlet, wherein the vent is for allowing an air to pass and enter or exit the first tube body and the inlet is for allowing a fluid from an external fluid source to enter the first tube body; and the first tube body having a closed bottom that sealably communicates with the exterior through a centrally formed outlet; a second tube body, being a cylinder-like container defined by a peripheral wall, the second tube body having a closed top that sealably communicates with the exterior through a vent and an inlet, wherein the vent is for allowing an air to pass and enter or exit the second tube body and the inlet is for allowing at least one part of the fluid from the outlet of the first tube body to enter the second tube body and the second tube body having a closed bottom that sealably communicates with the exterior through a centrally formed outlet; and a connecting pipe, being arranged between the outlet of the first tube body and the inlet of the second tube so as to connect the first tube body and a second tube body.

Further, the peripheral walls of the first tube body and the second tube body each extend downward beyond the bottom so the peripheral wall and the bottom form a pipe-receiving space around the outlet, and at least one of the connecting pipe and an extension pipe is configured to be at least partially coiled around the outlet of the tube body and received in the pipe-receiving space of tube body.

Further, in the closed cell separation operation apparatus, there is further an extension pipe attached to the outlet of the second tube body for connecting the second tube body to a further said first tube body of a further said closed cell separation operation apparatus.

Further, in the closed cell separation operation apparatus, at least one of the peripheral walls of the first tube body and the second tube body has a lower edge formed with a notch for the connecting pipe to pass therethrough, so that when the first tube body and the second tube body stands on a plane, an access to the pipe-receiving space of the first tube body and the second tube body is left for the connecting pipe to pass therethrough.

Further, in the closed cell separation operation apparatus, at least one of the connecting pipes and the extension pipe is equipped with a valve for closing or opening the pipe, and the valve is a tube clamp, a flow control valve, a multi-way tap connector or an on-off fluid valve.

Further, in the closed cell separation operation apparatus, wherein at least one of the bottoms of the first tube body and the second tube body has a downward conical structure with a diameter gradually reduces and reaches a smallest extent thereof at a periphery of the outlet of the first tube body and the second tube body.

Further, in the closed cell separation operation apparatus, at least one of the vents of the first tube body and the second tube body is equipped with a filter for filtering the air passing through the vent.

Further, in the closed cell separation operation apparatus, there is further an air-pressure source attached to at least one of the vents of the first tube body and the second tube body for pushing air into or drawing air from the first tube body and the second tube body.

The air-pressure source is an air pump or an air injector.

Further, in the closed cell separation operation apparatus, wherein at least one of the inlets and the vents of the first tube body and the second tube body is configured to be detachably sealed by a seal in an airtight manner.

Further, in the closed cell separation operation apparatus, at least one of the inlets and the vents of the first tube body and the second tube body is further provided with a pipe that is equipped with a vale.

The valve is a tube clamp, a flow control valve, a multi-way tap connector or an on-off fluid valve.

### BRIEF DESCRIPTION OF THE DRAWINGS

**Fig. 1** is perspective view of a closed cell separation operation apparatus according to one embodiment of the present invention;
**Fig. 2** through **Fig. 4** are applied, cross-sectional view of the closed cell separation operation apparatus of the present invention, illustrating the operation of the closed cell separation operation apparatus during centrifugation and post-centrifugation supernatant separation;
**Fig. 5** is a perspective view of an alternative implementation of the closed cell separation operation apparatus, wherein two closed cell separation operation apparatus of the present invention are connected in series; and
**Fig. 6** is a perspective view of a closed cell separation operation apparatus according to another embodiment of the present invention.

### DETAILED DESCRIPTION OF THE INVENTION

While a preferred embodiment provided hereinafter for illustrating the concept of the present invention has been described above, it is to be understood that the components of the embodiment shown in the accompanying drawings are depicted for the sake of easy explanation and need not to be made to exact scale.

For the descriptions herein, the singular forms "a", and "an" include plural referents unless the context clearly indicates otherwise.

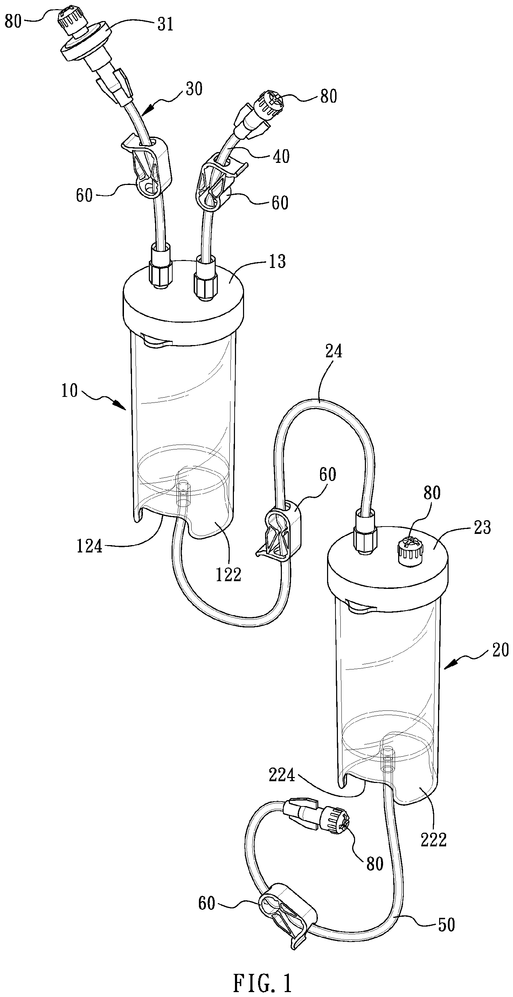

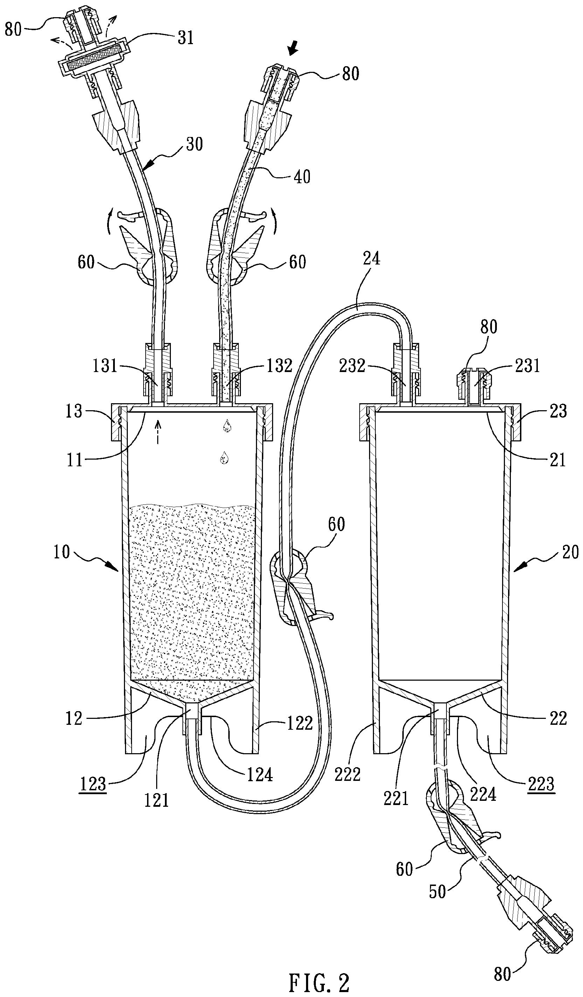

Referring first to **Fig. 1** and **Fig. 2****,** according to one embodiment of the present invention, a closed cell separation operation apparatus primarily comprises a first tube body **10** capped by a first cap **13**, a second tube body **20** capped by a second cap **23**, and a connecting pipe **24** connecting the two tube bodies **10**, **20.**

As the first tube body **10** and the second tube body **20** are substantively identical in terms of structure, the following description is directed to only one of them for succinctness. The first tube body **10** or the second tube body **20** is a cylinder-like container defined by a peripheral wall **122** or **222.** The first tube body **10** or the second tube body **20** has an open top fully open to the exterior through an opening **11** or **21** and a closed bottom **12** or **22** that sealably communicates with the exterior through a centrally formed outlet **121** or **221.** Particularly, the bottom **12** or **22** has a funnel-like, or downward conical structure with a diameter that gradually reduces and reaches its smallest extent at the periphery of the outlet **121** or **221.** The peripheral wall **122** or **222** of the tube body **10** or **20** extends downward beyond the closed bottom **12** or **22** so that it works with the funnel-like bottom **12** or **22** to form a pipe-receiving space **123** or **223** around the outlet **121** or **221.** The lower edge of the peripheral wall **122** or **222** is further formed with a notch **124** or **224**, so that when the first tube body **10** or the second tube body **20** stands on a plane, an access to the pipe-receiving space **123** or **223** is created. It is to be noted that the first tube body **10** or the second tube body **20** is sized similar to the existing centrifuge tubes or blood collection tubes, so as to be compatible with existing centrifuges.

Similarly, since the first cap **13**, a second cap **23** are substantively identical in terms of structure, the following description is directed to only one for succinctness. The first cap **13** or the second cap **23** is configured to cap the first tube body **10** or the second tube body **20** from top and has a vent **131** or **231** and an inlet **132** or **232.** As detailed below, each of the vent **131** or **231** and the inlet **132** or **232** is configured to be removably connected to pipes in used and/or detachably capped by a seal **80** in an airtight manner when not in use.

It is to be understood that while the present embodiment has the first tube body **10** or the second tube body **20** capped by the cap **13** or **23** formed with the vent **131** or **231** and the inlet **132** or **232**, the disclosed closed cell separation operation apparatus in another embodiment may have the vent **131** or **231** and the inlet **132** or **232** directly formed on a closed top surface of the first tube body **10** or the second tube body **20** instead of using the cap **13** or **23** to close the first tube body **10** or the second tube body **20.**

The connecting pipe **24** is arranged between the outlet **121** of the first tube body **10** and the inlet **232** of the second tube body **20** to connect the first tube body **10** and the second tube body **20** for liquid communication. The connecting pipe **24** can be partially coiled around the outlet **121** of the first tube body **10** to be well received in the pipe-receiving space **123** of the first tube body **10.** With the notch **124** formed on the peripheral wall **122** of the first tube body **10**, the connecting pipe **24** can extend between the first tube body **10** and the second tube body **20** without preventing the first tube body **10** from standing on any plane stably. The connecting pipe **24** is further equipped with a valve **60** for closing or opening the connecting pipe **24.**

In use, with the connecting pipe **24** closed by the valve **60,** a feeding pipe **40** communicated with a source of a fluid to be processed (not shown), such as a filled blood bag, a blood gas syringe or a specimen tube, is connected to the inlet **132** of the first tube body **10.** At this time, the vent **131** of the first tube body **10** remains open, so that the fluid to be processed (e.g., whole blood) can be transferred to the first tube body **10**, as shown in **Fig. 2****.** Herein, the vent **131** of the first tube body **10** may be further provide with a filter **31** for preventing foreign objects from entering the of the first tube body **10** and thereby ensuring a contamination-free operation environment. Specifically, as shown in **Fig. 2**, a venting pipe **30** equipped with a filter **31** is attached to the vent **131** of the first tube body **10.** The venting pipe **30** may be also opened or released by a valve **60.** After the fluid is filled into the first tube body **10**, the inlet **132** of the first tube body **10** can be capped using a seal **80** directly in an airtight manner, or the feeding pipe **40** remained connected with the inlet **132** of the first tube body **10** is capped using a seal **80** in an airtight manner, as shown **Fig. 1****.** Similarly, the vent **131** of the first tube body **10** can be capped using a seal **80** directly in an airtight manner, or the venting pipe **30** remained connected with the vent **131** is capped using a seal **80** in an airtight manner, as shown **Fig. 1****.**

It is to be noted that when the connecting pipe **24**, the venting pipe **30**, and the feeding pipe **40** are closed by their respective valves **60**, the interior of the first tube body **10** is sealed from the exterior and forms a closed environment. Also, the second tube body **20** is now with its vent **231** and outlet **221** sealed and isolated from the exterior.

Then the entire closed cell separation operation apparatus can be moved into a centrifuge (not shown) for centrifugation. Therein, the first tube body **10** and the second tube body **20** are seated in two adjacent places in the centrifuge, with the connecting pipe **24** partially coiled around the outlet **121** and received in the pipe-receiving space **123** of the first tube body **10.** This prevents the connecting pipe **24** from undesired excessive movement during centrifugation.

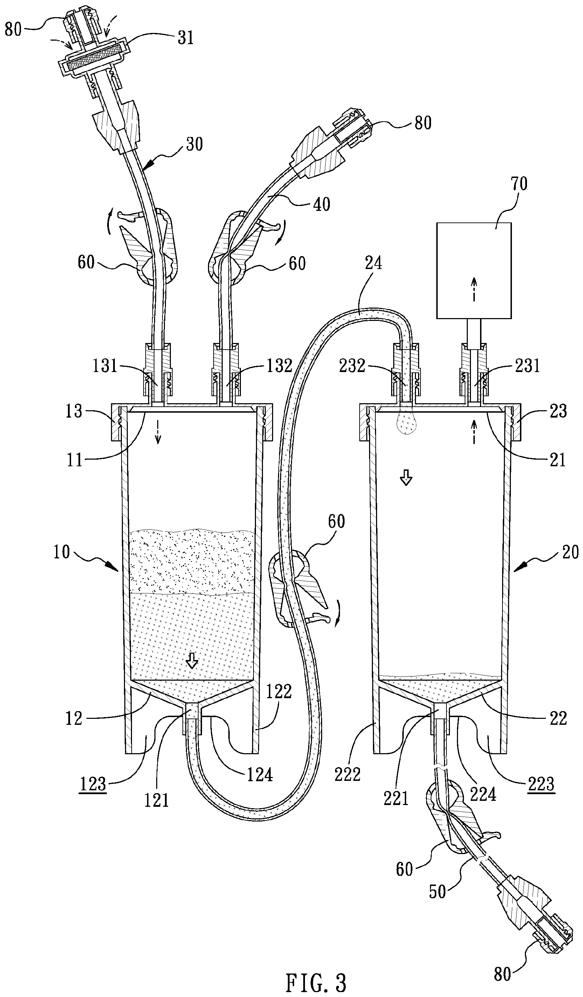

Now referring to **Fig. 3****,** the fluid in the first tube body **10** has separated into a supernatant (e.g., plasma for whole blood centrifugation) and a sediment (e.g., blood cells for whole blood centrifugation). Then, for the sediment to move into the second tube body **20**, the valve **60** on the connecting pipe **24** is open and the caps **80** are removed from the venting pipe **30** of the first tube body **10** and the vent **231** of the second tube body **20**, respectively. Optionally, an air-pressure source **70** may be removably connected to the vent **231** of the second tube body **20** for accelerated migration of the sediment with a drawing force.

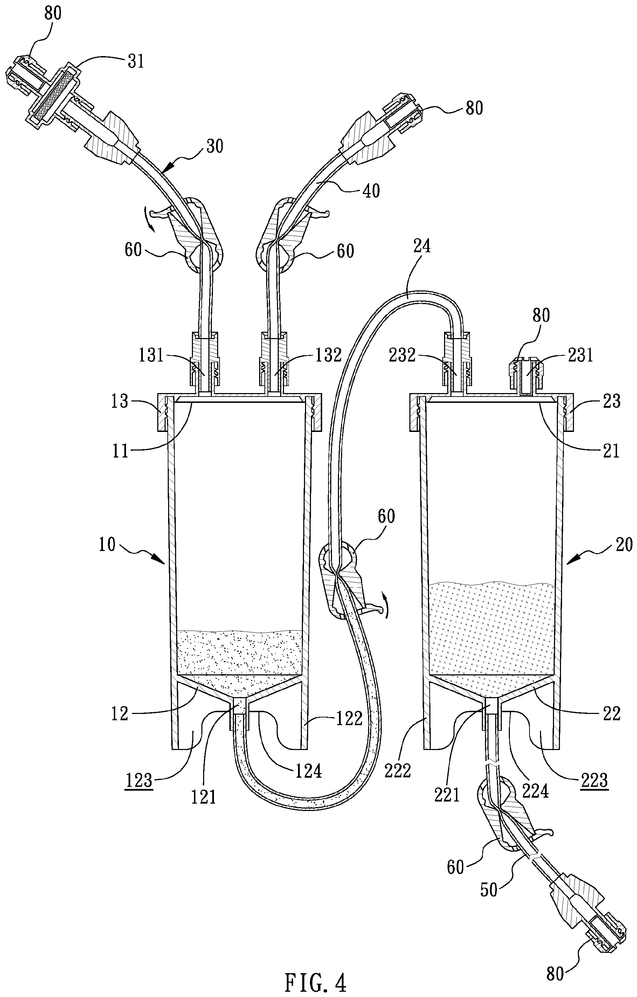

Since the bottom **12** of the first tube body **10** has the funnel-like, or downward conical, structure, and the outlet **121** is at the bottom-most position of the bottom **12** of the first tube body **10**, the migration of the sediment can be performed precisely without having the supernatant unintentionally move into the second tube body **20.** Besides, as the sediment directly flows into the second tube body **20**, the risk of spills can be completely eliminated. After the sediment enters the second tube body **20**, the connecting pipe **24** is closed by its valve **60** again, and the vent **231** of the second tube body **20** with the air-pressure source **70** having been detached is capped again, so as to seal the supernatant and the sediment in the first tube body **10** and the second tube body **20**, respectively. Consequently, all these enable effective and efficient isolation of the sediment form the supernatant, as shown in **Fig. 4****.**

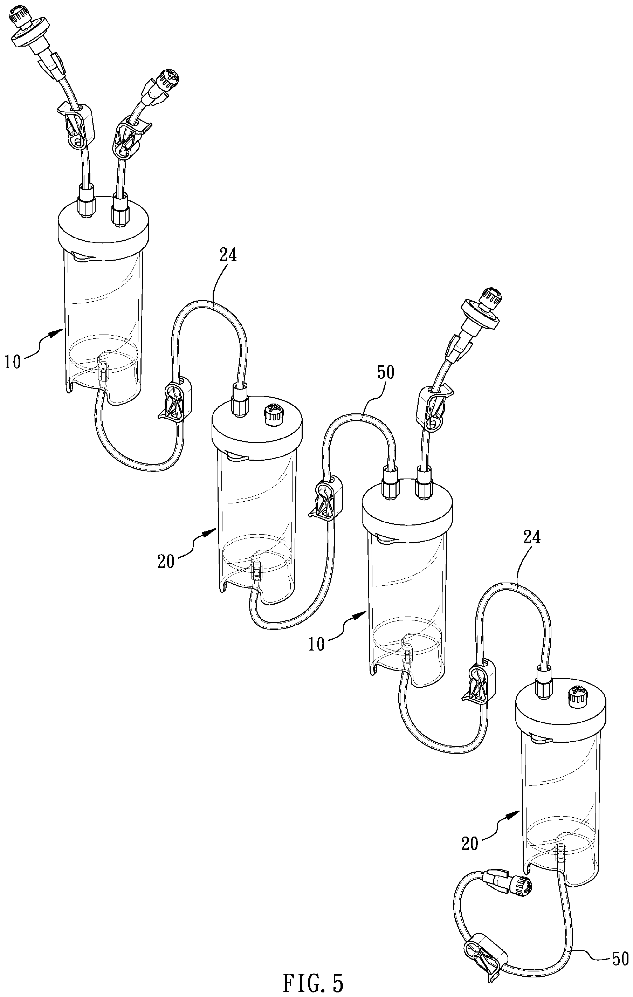

Referring to **Fig. 5**, for various applications where multiple separations are required, another closed cell separation operation apparatus of the present invention may be connected in series to the first closed cell separation operation apparatus. Specifically, an extension pipe **50** may be used to connect the first tube body **10** of the further closed cell separation operation apparatus. In this case, the extension pipe **50** may be coiled around the outlet **221** and received in the pipe-receiving space **223** of the second tube body **20** during centrifugation as described with respect to the connecting pipe **24.**

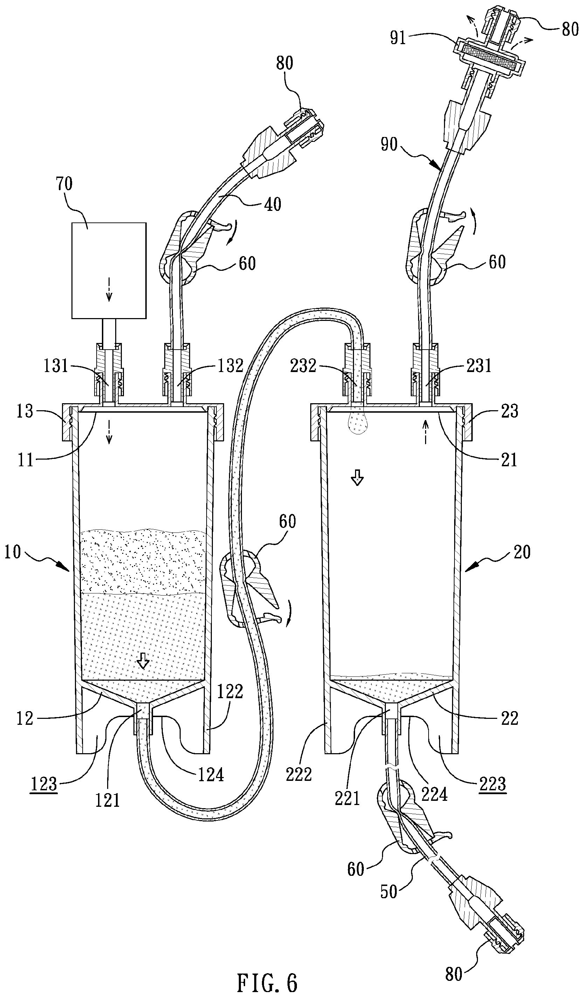

**Fig. 6** depicts another embodiment of the closed cell separation operation apparatus of the present invention. As shown in **Fig. 6**, the closed cell separation operation apparatus is further provided with a second venting pipe **90** attached to the vent **231** of the second tube body **20**. The second venting pipe **90** is equipped with a further valve **60** for closing or opening it. The second venting pipe **90** is connected to an additional filter **91** for preventing foreign objects from entering the second tube body **20** and further ensuring a contamination-free operation environment. In this case, an air-pressure source **70**, as described previously may be attached to the vent **131** of the first tube body **10** for pushing the content in the first tube body **10** into the second tube body **20.**

The foregoing valves **60** may each independently be a tube clamp, a flow control valve, a multi-way connector or an on-off fluid valve, without limitation.

The air-pressure source **70** attached to the vent **131** of the first tube body **10** and/or the vent **231** of the second tube body **20** may be an air pump or an air injector or any other device that can change the pressure in the first tube body **10** and the second tube body **20** to facilitate fluid flow from the first tube body **10** to the second tube body **20.**

At last, centrifugation is widely used in various medical and biological applications and well known to those skilled in the art, so the related operation and principle are not described in detail for the sake of succinctness. Likewise, the cell isolation technology involving in the use of the disclosed closed cell separation operation apparatus is also known in the art and not discussed herein.

With the configurations described above, the closed cell separation operation apparatus achieves the objectives of the present invention by providing a closed, contamination-free environment for both centrifugation and post-centrifugation supernatant separation while eliminating the risks of spills and the needs for costly laboratory equipment.

The present invention has been described regarding the preferred embodiments and it is understood that the embodiments are not intended to limit the scope of the present invention. Moreover, all modifications, equivalent changes or improvements which do not depart from the concept of the present invention should be encompassed by the appended claims.

## Claims

1. A closed cell separation operation apparatus, comprising:
first tube body (10), being a cylinder-like container defined by a peripheral wall (122), the first tube body (10) having a closed top that sealably communicates with the exterior through a vent (131) and an inlet (132), wherein the vent (131) is for allowing air to pass and enter or exit the first tube body (10) and the inlet (132) is for allowing fluid from an external fluid source to enter the first tube body (10); and the first tube body (10) having a closed bottom (12) that sealably communicates with the exterior through a central outlet (121);
a second tube body (20), being a cylinder-like container defined by a peripheral wall (222), the second tube body (20) having a closed top that sealably communicates with the exterior through a vent (231) and an inlet (232), wherein the vent (231) is for allowing an air to pass and enter or exit the second tube body (20) and the inlet (232) is for allowing at least one part of the fluid from the outlet (121) of the first tube body (10) to enter the second tube body (20) and the second tube body (20) having a closed bottom (22) that sealably communicates with the exterior through a centrally formed outlet (221); and
a connecting pipe (24), being arranged between the outlet (121) of the first tube body (10) and the inlet (232) of the second tube body (20), so as to link the first tube body (10) and the second tube body (20).

2. The closed cell separation operation apparatus of claim 1, wherein the peripheral wall (122) of the first tube body (10) extends downward the bottom (12) of the first tube body (10) so the peripheral wall (122) and the bottom (12) form a pipe-receiving space (123) around the outlet (121) of the first tube body (10).

3. The closed cell separation operation apparatus of claim 2, wherein the peripheral wall (122) of the first tube body (10) has a lower edge formed with a notch (124) for the connecting pipe (24) to pass therethrough.

4. The closed cell separation operation apparatus of claim 1, further comprising an extension pipe (50) attached to the outlet (221) of the second tube body (20) for connecting the second tube body (20) to a further said first tube body (10) of a further said closed cell separation operation apparatus.

5. The closed cell separation operation apparatus of claim 4, wherein the peripheral wall (222) of the second tube body (20) extends downward the bottom (22) of the second tube body (20) so the peripheral wall (222) and the bottom (22) form a pipe-receiving space (223) around the outlet (221) of the second tube body (20).

6. The closed cell separation operation apparatus of claim 5, wherein the peripheral wall (222) of the second tube body (20) has a lower edge formed with a notch (224) for the connecting pipe (24) to pass therethrough.

7. The closed cell separation operation apparatus of claim 1, wherein the connecting pipe (24) is equipped with a valve (60) for closing or opening the connecting pipe (24).

8. The closed cell separation operation apparatus of claim 4, wherein the extension pipe (50) is equipped with a valve (60) for closing or opening the extension pipe (50).

9. The closed cell separation operation apparatus of claim 2, wherein the bottom (12) of the first tube body (10) has a downward conical structure with a diameter gradually reduces and reaches a smallest extent thereof at a periphery of the outlet (121) of the first tube body (10).

10. The closed cell separation operation apparatus of claim 5, wherein the bottom (22) of the second tube body (20) has a downward conical structure with a diameter gradually reduces and reaches a smallest extent thereof at a periphery of the outlet (221) of the second tube body (20).

11. The closed cell separation operation apparatus of claim 1, wherein at least one of the vents (131, 231) of the first tube body (10) and the second tube body (20) is equipped with a filter (31) for filtering the air passing through the vent (131, 132).

12. The closed cell separation operation apparatus of claim 1, wherein at least one of the vents (131, 231) of the first tube body (10) and the second tube body (20) is equipped with an air-pressure source (70) for pushing an air into or drawing an air from the first tube body (10) and the second tube body (20).

13. The closed cell separation operation apparatus of claim 1, wherein at least one of the inlets (132, 232) and the vents (131,231) of the first tube body (10) and the second tube body (20) is configured to be detachably sealed by a seal (80) in an airtight manner.

14. The closed cell separation operation apparatus of claim 1, wherein at least one of the inlets (132, 232) and the vents (131,231) of the first tube body (10) and the second tube body (20) is further provided with a pipe that is equipped with a vale.
